# EUROPÄISCHE PATENTANMELDUNG

(11) **EP 1 561 823 A1**
(43) Veröffentlichungstag der Anmeldung: **10.08.2005**
(21) Anmeldenummer: 04090034.2
(22) Anmeldetag: 04.02.2004
(51) Int. Cl.: C12Q 1/68

(54) **Verfahren zum Nachweis von Einzelnukleotid-polymorphismen (SNP) in Genen des Arzneimittelmetabolismus und Testkit zur Durchführung des Verfahrens**

(71) Anmelder: Biotez Berlin-Buch GmbH, 13125 Berlin (DE)
(72) Erfinder: Neunaber, Ralf, Dr., 10999 Berlin (DE); Strohner, Pavel, Dr., 13187 Berlin (DE); Schreiber, Joachim, Dr., 13125 Berlin (DE); Voigt, Gesine, 10247 Berlin (DE)
(74) Vertreter: Baumbach, Friedrich, Dr.

(57) **Zusammenfassung**

Die Erfindung betrifft ein vereinfachtes Verfahren zum Nachweis von SNP in Cytochrom P450 (CYP)-Genen des Menschen und ein Test Kit mit folgenden Komponenten:
1. Neu entwickelte synthetische Oligonukleotide, die geeignet sind, Genabschnitte in Genen des Arzneimittelmetabolismus mit Hilfe der "DNA Polymerase Chain Reaction" Technologie (PCR) isoformspezifisch (IS-PCR) aus genomischer Leukozyten-DNA zu amplifizieren,
2. Komponenten, die es ermöglichen, einzelsträngig biotinierte IS-PCR Produkte auf Streptavidin beschichteten Mikrotiterplatten (SA-MTP) selektiv, thermostabil und einzelsträngig zu immobilisieren,
3. testoptimierte, allelspezifische, Fluoresceinisothiocyanat (FITC) markierte synthetische Oligonukleotide, die durch Hybridisierung, nachfolgende stringente Waschung und anschließende Detektion mittels Fluorometrie bzw. Photometrie eine sichere Genotypisierung der immobilisierten Amplifikationsprodukte (Amplikons) gewährleisten, oder alternativ dazu
4. Streptavidin beschichtete Glasobjektträger (SA-Chip) auf denen biotinierte, isoformspezifische, synthetische Oligonukleotide immobilisiert sind, welche mit partiell einzelsträngigen PCR Produkten hybridisiert werden, die durch diskriminierende Verlängerung eines allelspezifischen, endständig FITC markierten Oligonukleotides mittels Taq DNA Polymerase erzeugt wurden und durch nachfolgende stringente Waschung und anschließende Detektion mittels Fluorometrie bzw. Photometrie eine sichere Genotypisierung der hybridisierten Amplikons gewährleisten,
5. neu entwickelte synthetische Oligonukleotide, die geeignet sind, an PCR amplifizierten CYP Genabschnitten, mit Hilfe der "Taq DNA Ligase detection reaction" Technologie (LDR) SNP spezifische, mit einer synthetischen Erkennungssequenz (ZIP Code) versehene, Oligonukleotide diskriminierend mit daran anschließenden 3' FITC markierten Oligonukleotiden zu ligieren,
6. 3' Biotin markierte, auf Streptavidin beschichteten Polycarbonatobjektträgern (PC-Chips) immobilisierte, revers komplementäre, synthetische Oligonukleotide, die es ermöglichen, die Ligationsprodukte anhand ihrer ZIP Code Sequenz allespezifisch zu hybridisieren und anschließend mittels Fluorometrie bzw. Photometrie zu detektieren und so eine sichere Genotypisierung der hybridisierten Amplikons auch bei paralellem Nachweis von multiplen SNP's in einem Ansatz zu gewährleisten (Multiplex Ansatz).

Im Unterschied zu den bekannten "high-throughput"-Verfahren ermöglicht es die hier beschriebene Erfindung, auch an wenigen Einzelpatienten bei niedrigen Personal- und Sachkosten die therapeutisch wichtigen Befunde zur individuellen Ausstattung mit den verschiedenen CYP Allelen zu erheben.

Die in den Punkten 5 und 6 der Erfindung aufgeführten Merkmale ermöglichen es, an Einzelpatienten gleichzeitig mindestens 8 verschiedene CYP Allele in einem Multiplex Ansatz zu amplifizieren, mittels LDR zu typisieren und anschließend auf einem Multiplex-PC-Chip bei einer hohen Diskriminierung und damit diagnostischen Sicherheit, zu detektieren. Die Zeit-, Aufwands- und Materialeinsparung, sowie die höhere diagnostische Sicherheit dieses vereinfachten Verfahrens stellen die wesentlichen Vorteile gegenüber dem Stand der Technik dar.

## Beschreibung

Gegenstand der Erfindung ist ein Satz von Substanzen und Vorschriften - insgesamt wird ein solcher Satz auch als "Kit" bezeichnet - der es gestattet, bestimmte, für den Arzneimittelmetabolismus wichtige Polymorphismen in den Cytochrom P450 Genen (CYP) eines Menschen mit einfachen Mitteln zuverlässig nachzuweisen.
Anwendungsgebiete sind die Molekularbiologie und die Medizin, insbesondere die medizinische Diagnostik.

Polymorphismen in Genen des Arzneimittelmetabolismus stehen mit dem Fortschritt bei der Erforschung der Beziehungen zwischen genetischer Diversität und Arzneimittelunverträglichkeiten im Mittelpunkt des Interesses. Das gilt im besonderen für bestimmte Einzelnukleotidpolymorphismen ("single nucleotide polymorphisms"-SNP's) an Genen des sogenannten Biotransformationssystems. Ein großer Teil der heute verwendeten Medikamente, vor allem solche mit lipophilem Charakter, werden von den Enzymen der Phase I des Biotransformationssystems, den Cytochrom P450 Enzymen, metabolisiert [Gonzales, F. J., Pharmacological Reviews 40, (1989) 243-288; Guengerich et al., Journal of Biological Chemistry 266 (1991) 10019-10022]. Als Folge dieser enzymatischen Reaktionen werden je nach chemischer Struktur des Medikaments, nach Art und Genotyp des Cytochrom P450 Isoenzyms oder infolge nachgeschalteter sogenannter Phase II Enzyme (NAT I u. II, GSTM u. a.) [Brockmüller et al. Toxicology Letters 102-103 (1998) 173-183] die Medikamente entweder physiologisch aktiviert, oder sie werden inaktiviert und zur Ausscheidung vorbereitet. Polymorphismen an diesen Genen sind in der Bevölkerung relativ weit verbreitet und führen zu erheblichen individuellen Unterschieden in den Umsatzraten der betroffenen Arzneimittel. In der Konsequenz können je nach Medikament und Allel individuelle Über- oder Unterdosierungen die Folge sein. Es können sich unerwünschte Nebenwirkungen einstellen, oder aber eine Wirkung kann ganz ausbleiben [Wormhoudt et al. Critical Reviews in Toxicology 29 (1999) 59-124; Ingelman-Sundberg M. Toxicology Letters 102-103 (1998) 155-160]. Die genaue Kenntnis der genetischen Prädisposition eines Patienten zu solcherart verändertem Arzneimittelstoffwechsel ist dementsprechend für seine individuelle Arzneimitteltherapie von entscheidender Bedeutung. Als Folge konsequenter Genotypisierung von Patienten mit erhöhtem Risiko (z.B. von chronisch kranken Patienten, oder von solchen mit einer entsprechenden Familienanamnese) sind erheblich verminderte Nebenwirkungsrisiken, Kosteneinsparungen, aber auch völlig neuartige Medikationsstrategien denkbar. Praktische Voraussetzung für eine konsequente Genotypisierung vieler einzelner Patienten ist jedoch ein kostengünstiges und zuverlässiges Genotypisierungsverfahren.

Die Cytochrom P450 Enzyme werden artübergreifend anhand ihrer Aminosäuresequenzhomologie in Familien (Homologie >40% - bezeichnet mit einer arabischen Ziffer) und Subfamilien (Homologie >55% - bezeichnet mit einem Großbuchstaben) unterteilt. Zum Beispiel ist CYP2C9 ein Cytochrom der Familie 2, Subfamilie C, Isoform Nr. 9 [Nelson et al. Pharmacogenetics 6 (1996) 1-42]. Die zum Teil extrem hohe Sequenzhomologie bei Genen einer Subfamilie muß bei der Entwicklung von Genotypisierungsverfahren, die auf einer Amplifikation von Genteilsequenzen beruhen, denn auch vorrangig berücksichtigt werden. So sind die Pseudogene CYP2D7 und CYP2D8 zu >90% identisch mit CYP2D6. Ein sicherer Nachweis einzelner Allele setzt somit eine sichere Differenzierung der homologen Gene (Isoformen) voraus. Technisch ist dies durch diskriminierende PCR Amplifikationsverfahren möglich.

Zu den am besten charakterisierten und für den Arzneimittelmetabolismus bedeutendsten Cytochromen gehören CYP2C9, CYP2C19, CYP2D6 und CYP3A4 mit ihren diversen Allelvarianten. [Zu Übersicht und neuestem Stand der bekannten Polymorphismen menschlicher Cytochrom P450 Enzyme siehe [http://www.imm.ki.se/CYPalleles/]. Diese vier Isoenzyme zusammen setzen nach Schätzungen von Experten mehr als zwei Drittel aller wirksamen Bestandteile der heute gängigen Medikamente um. [Eine übersichtliche Zusammenstellung der umgesetzten Pharmazeutika findet sich unter [http://medicine.iupui.edu/flockhart/]. Wie in Tabelle 1 dargestellt ist, sind in der kaukasischen Bevölkerung die Allele CYP2C9*2, CYP2C9*3, CYP2C19*2, CYP2D6*3, CYP2D6*4, 2D6*5, 2D6*6 sowie weitere, weniger gut untersuchte Allele von CYP2D6 von größter Bedeutung [Wormhoudt et al. Critical Reviews in Toxicolgy 29 (1999) 59-124; Ingelman-Sundberg M. Toxicology Letters 102-103 (1998) 155-160; Marez et al. Pharmacogenetics 7 (1997) 193-202; De Morais et al. Mol. Pharmacol. 46 (1994) 594-598; Sachse et al. Am. J. Hum. Genet. 60 (1997) 284-295; Wrighton SA and Stevens JC. Crit. Rev. Toxicol. 22 (1992) 1-21; Cholerton et al. Trends Pharmacol. Sci. 13 (1992) 434-439; Saxena et al. Hum. Mol. Genet. 3 (1994) 923-926; Steen et al. Hum. Mol. Genet. 4 (1995) 2251-2257].

**Tabelle 1**

| Wichtige allele Varianten humaner Cytochrom P450 Isoenzyme, ihre Enzymaktivität und ihre Häufigkeit (%) in der kaukasischen Bevölkerung | | | |
|---|---|---|---|
| Allel | Enzymaktivität | Häufigkeit | Referenz |
| CYP2C9*2 | Vermindert | 16 | Ingelman-Sundberg (1998) |
| CYP2C9*3 | Vermindert | 6 | Ingelman-Sundberg (1998) |
| CYP2C19*2 | Keine | 10 | Ingelman-Sundberg (1998) |
| CYP2D6*3 | Keine | 2 | Sachse et al. (1997) |
| CYP2D6*4 | Keine | 23 | Marez et al. (1997) |
| CYP2D6*5 | Keine | 2-5 | Steen et al. (1995), Sachse et al. (1997) |
| CYP2D6*6 | Keine | 1-2 | Saxena et al. (1994), Sachse et al. (1997) |

Die klinische Relevanz der allelen Varianten von CYP3A4 ist zur Zeit Gegenstand intensiver Forschung [Eiselt et al. Pharmacogenetics 11 (2001) 447-458; Kuehl et al. Nature Genetics 27 (2001) 383-391; Westlind et al. Biochem. Biophys. Res. Comun. 259 (1999) 201-205].
Der Nachweis alleler Varianten der am Arzneimittelmetabolismus beteiligten Cytochrom P450 Isoenzyme erfolgte anfänglich mit Hilfe des Sequenzvergleichs entsprechender isolierter cDNA's, oder durch direkte Sequenzierung von aus genomischer DNA gewonnenen PCR Fragmenten [Sullivan-Klose et al. Pharmacogenetics 6 (1996) 341-349; Wang et al. Pharmacogenetics 5 (1995) 37-42 ; Inoue et al. Jpn. J. Hum. Genet. 39 (1994) 337-343]. Durch heterologe Expression vorsequenzierter oder modifizierter cDNA's in geeigneten Systemen und anschließende *in vitro* Charakterisierung konnte dann oft ein Zusammenhang zum Arzneimittelmetabolismus belegt werden [Haining et al. Arch. Biochem. Biophys. 333 (1996) 447-458]. Ein weiteres Verfahren zur Identifikation neuer SNP's - das SSCP ("single strand conformational polymorphisms") Verfahren - nutzt die sequenzabhängig unterschiedliche Mobilität von einzelsträngigen PCR Produkten in Harnstoff-Polyacrylamidgelen zum Nachweis von Mutationen [Stubbins et al. Pharmacogenetics 6 (1996) 429-439; Daly et al. Methods Enzymol. 272 (1996) 199-210].
Das zur Zeit neben der Sequenzierung am häufigsten genutzte Verfahren zum Nachweis von SNP's ist die "Restriktions-Fragment-Längen-Polymorphismus" (RFLP) Analyse [Sullivan et al. Pharmacogenetics 6 (1996) 341-349; Wang et al. Pharmacogenetics 5 (1995) 37-42]. Durch Einführung der MADGE (microplate array diagonal gel electrophoresis) Technologie [Day and Humphries Anal. Biochem. 222 (1994) 389-395] wurde es möglich, RFLP oder ARMS (amplification refractory mutation system) [Newton et al. Nucleic Acid Res. 17 (1989) 2503-2516) im 96 Kammer (96 well) Format durchzuführen. Ein in der Vergangenheit oft eingesetztes Verfahren für den Nachweis von SNP's ist der sogenannte "Southern blot". Eine Weiterentwicklung dieses Prinzips sind die zur Zeit noch in der Entwicklung befindlichen "Microarray" Gen Chip Verfahren. Beide Verfahren beruhen auf einer allelspezifischen Hybridisierungsreaktion an immobilisierten PCR Fragmenten, oder Detektionssonden. Im Falle des "Microarray" Verfahrens sind die Areale der Hybridisierung durch spezielle Belegungsverfahren zum Teil nur wenige nm im Durchmesser. Ein anderes, zunehmend eingesetztes, Verfahren zum Nachweis von SNP's macht von der "matrix-assisted laser desorption/ionization time-of-flight mass spectrometry" (MALDI/TOF-MS) Gebrauch. Dieses Verfahren setzt jedoch ebenfalls eine isoformspezifische PCR, eine anschließende Aufreinigung und den Allelnachweis mittels z.B. hybridisierter, synthetischer Oligonukleotide und den nachfolgenden, allelspezifischen Einbau zusätzlicher Nukleotide durch eine DNA Polymerase voraus.
Das Sequenzierverfahren ist vergleichsweise sehr aufwendig und insbesondere für die Identifizierung neuer SNP's geeignet. Es erfaßt aber weder Duplikationen noch Deletionen kompletter Gene. Das SSCP Verfahren kann zwar beides erfassen und ist daher für die frühe Identifikation von SNP's das Verfahren der Wahl, erfordert jedoch ebenfalls einen hohen Arbeitsaufwand, bzw. einen hohen Automatisierungsgrad und verursacht somit hohe Kosten. Das RFLP Verfahren macht oft mangels geeigneter Restriktionsorte den Austausch zusätzlicher Nukleotide in der Umgebung der alleldeterminierenden Position mittels PCR nötig. Auch ist der Verdau in einigen Fällen nur unvollständig und erfordert eine zusätzliche Validierung mittels Positivkontrolle bzw. Kreuzvergleich mit anderen Allelen. Die auf der allelspezifischen Hybridisierung aufbauenden Verfahren (TaqMan™; Dash™; GeneChip®; Code Link™) stellen eine echte Alternative für den Nachweis bekannter SNP's dar. Im Falle der "Microarray" (GeneChip®; Code Link™) Technologie entsteht jedoch ein besonderes Problem aus dem Anspruch dieser Technologieplattform, große Mengen an Sequenzdaten von nur einem Chip zu erhalten. Mit der Menge steigt die Schwierigkeit, ein gemeinsames Regime für alle auf dem Chip nachzuweisenden Allele zu etablieren. Es werden umfangreiche Kontrollen sowie aufwendige und komplizierte Auswertesysteme erforderlich und somit steigen die Kosten. Für die klinische Diagnostik, sowie für klinische Studien mit vordefinierten, eingegrenzten, objektspezifischen Allelen bietet sich somit eher ein sogenannter "low density Chip" in Form eines Glasobjekträgers mit 32 bis maximal 384 möglichen spezifischen Arealen (spots) an.

Zur Zeit werden Komplettlösungen für den Nachweis von SNP's in Arzneimittel metabolisierenden Genen nur von wenigen Firmen angeboten:
- TaqMan Technologie (Applied Biosystems) - Prinzip: Fluoreszenzdetektion mittels mittelständiger Hybridisierungssonde. Der Nachweis des Allels geschieht über die 5'-3' Exonucleaseaktivität einer DNA Polymerase, welche an perfekt passenden Primern gequenchte 5'endständige Fluorophore (FAM und VIC™) freisetzt. Fehlgepaarte Primer werden von der DNA Polymerase unter den gewählten Bedingungen vollständig intakt (also gequencht) freigesetzt, während richtig gepaarte Primer im ungequenchten, fluoreszierenden Zustand freigesetzt werden. Für CYP2C9*2 und *3; CYP2C19*2 und *3; CYP2D6 * 3, *4, *6, *7 und *8 wird je ein kompletter Kit angeboten. Voraussetzung für den Einsatz dieser Technologie ist jedoch die Anschaffung eines TaqMan Gerätes, sowie der dazugehörigen Zusatzausrüstung.
- Der GeneChip CYP450 Assay (Affimetrix) beruht auf einer Hybridisierungsreaktion von allelspezifischen, endständig auf einem "Microarray Chip" immobilisierten Sonden mit Fluorophor markierten Amplikons. Das markierte Amplikon leitet sich aus einer vorgeschalteten Multiplex PCR ab, die über intronlokalisierte Primer eine isoformspezifische Amplifikation gewährleisten soll. Angeboten werden CYP2C9 *2 und *3, sowie 10 Allele des Gens CYP2D6 (von Exon 1-9) als Komplettansätze. Voraussetzung für den Einsatz dieser Technologie ist jedoch die Anschaffung des sehr kostenintensiven GeneChip Instrument Systems, sowie der dazugehörigen GeneChips, Reagenzien - und Primer Kits.
- Das Code Link™ P450 Verfahren (Motorola) basiert auf demselben Nachweisprinzip wie der Affimetrix Chip, setzt jedoch den Nachweis von 75 CYP450 SNPs (CYP2D6, -2C19, -1A1, 1-A2, -2E1, -3A4 und -1B1) auf einem Glasobjektträger um. Es erfordert ebenfalls die zusätzliche Anschaffung der sehr kostenintensiven Instrumentierung sowie natürlich der dazugehörigen Reagenzien und Primer Kits.
- Der Pharm-O-Kin Chip von GenScan Diagnostics ist ein Genchip auf der Basis eines Glasobjektträgers und weist 36 SNP's verschiedene P450 Isoenzyme sowie andere für den Arzneimitteltransport relevante Gene nach (CYP2D6, CYP2C9, CYP2C19, sowie NAT2 und MDR1).

Andere Systeme sind prinzipiell ebenfalls geeignet, Polymorphismen an Arzneimittel metabolisierenden Genen nachzuweisen. Die Oligonukleotidprimer für die PCR und/oder die Alleldetektion mit den dazugehörigen Reportersystemen müssen jedoch vom Anwender gesondert entwickelt werden. Solche Systeme sind:
- das DASH™ System von Hybaid, beruhend auf der Immobilisierung einzelsträngiger Amplikons auf SA-MTP und anschließender kinetischer Analyse der Schmelzpunkterniedrigung allelspezifischer Hybridisierungssonden. Als Reportersystem wird dabei der interkalierende Fluorophor SYBR Green™ verwendet. Voraussetzung ist die Anschaffung eines relativ kostenintensiven DASH™ Gerätes,
- das Pyrosequencing System von Pyrosequencing. Bei diesem Verfahren werden in einzelsträngig vorgelegte PCR Produkte, beginnend an einem spezifischen Primer, Einzelnukleotide sequentiell eingebaut. Der erfolgreiche Einbau eines Nukleotides wird dabei über eine Sulfurylase - Luciferase vermittelte Lichtemissionsreaktion detektiert. Überschüssiges Nukleotid wird vor der Zugabe eines weiteren Nukleotids durch Apyraseaktivität abgebaut. Durch sequentiellen Durchlauf aller Nukleotide wird somit ein kurzes bis mittel langes Sequenzieren in 96 well Platten realisiert. Die Technologie setzt die Anschaffung eines eigens für diesen Zweck entwickelten Gerätes, sowie der benötigten Reagenziencocktails voraus und ist daher vergleichsweise kostenintensiv und nur beschränkt einsetzbar,
- das SNP-IT System von Orchid stellt ein Gerätesystem dar, daß explizit für den Mittel- bis Hochdurchsatz von SNP-Nachweisen an PCR Amplikons entwickelt wurde. Es besteht im wesentlichen aus einem Roboter, welcher automatisch die Aufarbeitung der Proben, den Nachweis der Allele (dafür sind unterschiedlichste Technologien wie Gene Chip Hybridisierungsassay, Sequenzierung etc. integrierbar) und die Datenanalyse (nebst Statistik) leisten soll. Für diese aufwendigen Geräte sind die Anschaffungskosten entsprechend hoch.
- das SureScore™ System von Invitrogen ist ein auf 96 well Platten basierendes SNP Nachweissystem. Basistechnologie ist der spezifische Einbau markierter (Biotin bzw. FITC) Didesoxynukleotide an einer einzelstängigen, komplementären PCR Produktmatrix durch eine DNA Polymerase an der SNP Position. Der anschließende Nachweis geschieht durch einen markerspezifischen Antikörper, der über eine gekoppeltes Enzym eine Farbreaktion katalysiert.

Die Entwicklung von zuverlässigen Genotypisierungsverfahren, die durch geringen Zeit- und Geräteaufwand und relativ niedrige Kosten gekennzeichnet sind, ist dringend geboten, um eine intensivere klinische Erforschung der Zusammenhänge zwischen Arzneimittelstoffwechsel und Genotyp der beteiligter Enzyme und die Entwicklung hochgradig zuverlässiger und zugleich kostengünstiger, diagnostischer Verfahren zu ermöglichen.

Aus der Erfindung DE 102 37 691.3-41 ergab sich nach weiterer Entwicklung der Multiplexverfahren das Problem der relativ geringen Diskriminierung bestimmter SNP spezifischer Basenfehlpaarungen in Abhängigkeit zum umliegenden Sequenzkontext. Bei einigen Allelnachweisen war daher eine Multiplex-Detektionsreaktion (der paralelle Nachweis mehrerer Allele in einem Ansatz unter einem Regime) nur mit entsprechend schlechtem Signal/Rauschverhältnis realisierbar, was zu einer diagnostischen Unsicherheit beim Nachweis des betreffenden Allels führte. Zu diesem Zwecke wurde die bereits mehrfach aufgrund ihrer hohen Diskriminierung und gleichzeitigen Unabhängikeit vom Sequenzkontext beschriebene Ligase Detektions Reaktion (LDR) mittels thermostabiler Taq Ligase [Barani, F., Proc. Natl. Acad. Sci. USA 88, (1991)189-193] für den CYP SNP Nachweis angepaßt. Gleichzeitig konnte dieser Nachweisschritt mit einer allelspezifischen 5' Markierung des 3' FITC markierten Ligationsprodukts gekoppelt werden. Die so entstehenden, mit einer allspezifischen, am 5' Ende liegenden, ZIP Code Sequenz von 24 Basenpaaren ausgestatteten, Ligationsprodukte konnten in der Folge erfolgreich in einem Regime und Ansatz an 3'-Biotin markierte, revers komplementäre Oligonukleotide hybridisiert werden. Diese waren vorher definiert an Steptavidin vorbeschichtete PC-Chips immobilisiert worden und erlauben somit durch nachgeschaltete Waschung und Fluoreszenz bzw. gekoppelte kolorimetrische Detektion eine eindeutige und hochgradig diskriminierende Identifikation der nachzuweisenden CYP Allele.

Der Erfindung liegt demzufolge die Aufgabe zugrunde, ein solches Verfahren zu entwickeln und gleichzeitig ein Testkit zur Durchführung des Verfahrens zur Verfügung zu stellen

Die Erfindung wird gemäß den Ansprüchen realisiert. Sie betrifft ein vereinfachtes Verfahren zum Nachweis von SNP in CYP-Genen des Menschen und einen Test Kit mit folgenden Komponenten:
1. Neu entwickelte synthetische Oligonukleotide, vorzugsweise Oligonukleotide der Anlage 1, die geeignet sind, Genabschnitte in Genen des Arzneimittelmetabolismus mit Hilfe der "DNA Polymerase chain reaction" Technologie (PCR) isoformspezifisch (IS-PCR) aus genomischer Leukozyten-DNA zu amplifizieren,
2. Komponenten, die es ermöglichen, einzelsträngig biotinierte IS-PCR Produkte auf Streptavidin beschichteten Mikrotiterplatten (SA-MTP) selektiv, thermostabil und einzelsträngig zu immobilisieren,
3. testoptimierte, allelspezifische, Fluoresceinisothiocyanat (FITC) markierte, synthetische Oligonukleotide, vorzugsweise Oligonukleotide der Anlage 1, die entweder durch Hybridisierung, oder alternativ dazu durch diskriminierende Verlängerung einzelsträngiger PCR Produkte mittels Taq-Polymeraseaktivität, nachfolgende stringente Waschung und anschließende Detektion mittels Fluorometrie bzw. Photometrie eine sichere Genotypisierung der hybridisierten Amplikons gewährleisten, oder
4. alternativ zu SA-MTP's - aus Streptavidin beschichteten Glasobjektträgern (SA-Chip) auf denen biotinierte, isoformspezifische, synthetische Oligonukleotide immobilisiert sind, welche mit partiell einzelsträngigen PCR Produkten hybridisiert werden, die durch diskriminierende Verlängerung eines allelspezifischen, endständig FITC markierten Oligonukleotides mittels Taq DNA Polymerase erzeugt wurden und durch nachfolgende stringente Waschung und anschließende Detektion mittels Fluorometrie bzw. Photometrie eine sichere Genotypisierung der hybridisierten Amplikons gewährleisten,
5. neu entwickelte synthetische Oligonukleotide, die geeignet sind, an PCR amplifizierten CYP Genabschnitten, mit Hilfe der "Taq DNA Ligase detection reaction" Technologie (LDR) SNP spezifische, mit einer synthetischen Erkennungssequenz (ZIP Code) versehene, Oligonukleotide diskriminierend mit daran anschließenden 3' FITC bzw. 3' Cy5 markierten Oligonukleotiden zu ligieren,
6. 3' Biotin markierte, auf Streptavidin beschichteten PC-Chips immobilisierte, revers komplementäre, synthetische Oligonukleotide, die es ermöglichen, die Ligationsprodukte anhand ihrer ZIP Code Sequenz allespezifisch zu hybridisieren und anschließend mittels Fluorometrie bzw. Photometrie zu detektieren und so eine sichere Genotypisierung der hybridisierten Amplikons auch bei paralellem Nachweis von multiplen SNP's in einem Ansatz gewährleisten (Multiplex Ansatz).

Durch zusätzliche positive Nachweise der Deletion des CYP2D6 Gens (Allel: CYP2D6*5) und der zum Teil mehrfachen Duplikation desselben Gens (Allel: CYP2D6*2XN) mittels "long distance" PCR mit biotinierten Sense - und FITC markierten Antisense Primern und anschließendem Nachweis, des auf SA-MTP gebundenen Duplexes, wird somit eine Abschätzung des phänotypisch relevanten Genotyps bezüglich der wichtigsten Arzneimittel metabolisierenden Cytochrom P450 Enzyme ermöglicht.

Im Unterschied zu den bekannten "high-throughput"-Verfahren ermöglicht es die hier beschriebene Erfindung, auch an wenigen Einzelpatienten bei niedrigen Personal- und Sachkosten die therapeutisch wichtigen Befunde zur individuellen Ausstattung mit den verschiedenen CYP Allelen zu erheben.

Die Erfindung nutzt hochgradig genspezifische Oligonukleotidprimer, vorzugsweise Oligonukleotide der Anlage 1, für flankierende Regionen der allelbestimmenden Genabschnitte, um diese aus einem Hintergrund von sehr stark homologen Cytochromen der gleichen Subfamilie mittels diskriminierender PCR bzw. Multiplex-PCR zu amplifizieren. Der folgende Allelnachweis kann mit zum Teil unterschiedlichen Verfahren erfolgen:
1. durch Markierung eines Primers pro alleltragendem Genfragment mit Biotin; Amplifikation allelbestimmender Genabschnitte mit Oligonukleotidprimern gemäß Anlage 1 mittels diskiminierender PCR; anschließende Kopplung der markierten Amplikons an eine thermostabile Streptavidin-Mikrotiterplatte (SA-MTP), vorzugsweise eine SA-MTP der Firma BioTeZ (Patentnummer: DE10020885 A). Die verunreinigende genomische DNA sowie die Komplementärstränge werden anschließend durch stringentes Waschen entfernt; die dann einzelsträngig vorliegenden Amplikons mit allelspezifischen, FITC markierten Oligonukleotiden hybridisiert und danach erneut einer stringenten Waschung unterzogen.
2. durch asymmetrische PCR zur Erzeugung überschüssiger ssDNA Amplikons, anschließende Hybridisierung mit einem 5'-FITC markiertem Primer, dessen 3'-Ende mit der Position der Mutation korrespondiert, und nachfolgende, allelspezifische Verlängerung des markierten Primers durch diskriminierende Taq-Polymeraseaktivität mit dNTP's zu partiell einzelsträngigen Duplexen. Biotinierte genspezifische Oligonukleotide werden auf definierten Spots eines mit Streptavidin beschichteten Glasobjektträgers immobilisiert. Die partiell einzelsträngigen Duplexe werden nachfolgend mit den immobilisierten, genspezifischen Oligonukleotiden hybridisiert und durch stringente Waschung von nicht verlängerten, 5'-FITC markierten Oligonukleotidprimer befreit.
3. durch Überführen eines Aliquots des entstandenen, unmarkierten PCR Produkts in einen Multiplex LDR Ansatz bestehend aus:
   a.) neu entwickelten, mit synthetischen Erkennungssequenzen von 24 Basenpaaren Länge am 5' Ende markierten, allespezifischen SNP Oligonukleotidsonden, bevorzugt Oligonukleotiden aus Anlage 2,
   b.) isoformspezifischen, 5' phosphorylierten, 3' Fluoreszenz markierten, synthetischen Oligonukleotiden, deren 5' Ende unmittelbar dem nachzuweisenden, isoformspezifischen SNP folgt,
   c.) rekombinante DNA Ligase aus Thermophilus aquaticus in einem Nikotinamid Adenin Dinukleotid (NAD) haltigem Puffer,
   nachfolgende LDR in einem handelsüblichen Thermcycler,
   Hybridisierung der entstandenen, 3' Fluoreszenz markierten LDR Produkte an, zu den Erkennungssequenzen revers komplementären, 3' Biotin markierten, synthetischen Oligonukleotiden, welche zuvor an Streptavidin beschichteten PC-Chips immobilisiert worden waren in einer abgedichteten, temperierten und die Lösung bewegenden Inkubationskammer über dem Chip,
   Nachweis der allelrepräsentierenden 3'-FITC markierten Oligonukleotide in Lösung über dem PC-Chip durch anschließende Anti-FITC-Ak-HRP Kopplung, Waschung und kalorimetrischen Nachweis mittels eingefüllter TMB Reagenz. Besagte TMB Reagenz fällt in Ruhe in den die Anti-FITC-Ak-HRP tragenden Spots aus. Alternativ dazu durch direkte Laser enhanced Fluorometrie bei Einsatz einer 3' Cy5 Fluoreszenzmarkierung.

Der Nachweis der allelrepräsentierenden 5'-FITC markierten Oligonukleotide geschieht in den Verfahren 1 und 2 durch anschließenden Anti-FITC-Ak-HRP Elisa.

Im Verfahren gemäß Punkt 3 wird als interne Kontrolle eine Probe bestehend aus gentechnisch gewonnener Plasmid DNA (pDNA), vorzugsweise der Plasmid DNA-Sequenz aus Anlage 2, welche allelspezifische Fragmente von pUC 19 Genabschnitten enthält, mitgeführt.

Dieses hochempfindliche Verfahren erlaubt den sicheren Nachweis der im Anhang und in den Ausführungsbeispielen aufgeführten Allele unter Einsatz von Laborstandardgeräten und/oder handelsüblichen Durchlichtscannern.

Die erfindungsgemäße Vereinfachung in dem hier beschriebenen Verfahren besteht darin, daß
1. die erfindungsgemäß eingesetzten Primer und Sonden einen hochselektiven Allel-Nachweis an genomischer DNA innerhalb weniger Stunden ermöglichen, und
2. der Test als multipler Assay durchgeführt wird, in dem eine Mehrzahl von einzelnen Allelen in einem speziell angepaßten Protokolldurchlauf auf einer MTP, bzw. mehreren Glasobjekträgern, gleichzeitig erfaßt werden, und
3. für den Fall des Nachweises auf einer MTP nur solche Geräte zum Einsatz kommen, die zur Standardausrüstung eines mit PCR bzw. Elisa Methoden befaßten Labors gehören, und
4. im Falle des Nachweises auf Glasobjekträgern, durch Vorschaltung einer automatisierten Übertragung der IS-PCR Produkte, eine wesentliche Verringerung des personellen und zeitlichen Aufwands, des einzusetzenden Probenmaterials, sowie die Minimierung möglicher Belegungsfehler erzielt werden, und
5. die erfindungsgemäß eingesetzten synthetischen Oligonukleotide des LDR Verfahrens einen hochselektiven Nachweis multipler CYP Allele eines Probanden aus genomischer DNA innerhalb weniger Stunden aus drei aufeinander folgenden Ansätzen ermöglichen, und
6. die Hybridisierung der LDR Produkte zum Nachweis der getesteten Allele in einem "ein Kammer" System durchgeführt werden kann und dadurch Materialbedarf, Pipettieraufwand und damit verbunden auch die Fehlerrate im Vergleich mit DE 102 37 691.3-41 weitergehend deutlich herabsetzt werden, und
7. die Alleldifferenzierung im LDR Verfahren mit einem z.T. erheblich gesteigerten Signal/Rausch Verhältnis erfolgen kann und somit die diagnostische Sicherheit in diesem Multiplex Assay ebenfalls deutlich verbessert wird, und
8. die entwickelten synthetischen Erkennungssequenzen (ZIP Code Sequenzen), sowie deren revers komplemtäre Pendants einen universell anwendbaren durch Sequenzvergleich mit der humanen Genomdatenbank in ihrer nicht vorhandenen Interaktion mit dem menschlichen Genom abgesicherten Code darstellen, der zur Identifizierung auch anderer humaner SNP's mittels der LDR bzw. Ligase Ketten Reaktion (LCR) geeignet ist und eine universelle Platform für den multiplen SNP Nachweis darstellt.

Als interne Kontrollen können Proben bestehend aus gentechnisch gewonnener Plasmid DNA (pDNA), vorzugsweise der Plasmid DNA-Sequenzen der Anlage 1, welche allelspezifische Fragmente von CYP-Genabschnitten enthalten, mitgeführt werden.

Die Erfindung soll nachfolgend durch Ausführungsbeispiele näher erläutert werden.

### AUSFÜHRUNGSBEISPIELE

### Beispiel 1

Isoformspezifische Amplifikation eines Genabschnitts von CYP2C9 aus genomischer Leukozyten DNA mittels PCR
a) Isolation der genomischen DNA (gDNA)
Genomische DNA kann nach bereits beschriebenen Standardverfahren aus Leukozyten des menschlichen Blutes isoliert werden (Vogelstein B. and Gillespie D. PNAS, (1979) 76: 615-9). Hierbei sollte eine gDNA Konzentration von 5-50 ng/µl angestrebt werden, um bei Einsatz von 0,5-1 µl gDNA/PCR Ansatz eine ausreichende Kopienzahl vorzulegen. Der Qualität der gDNA kann bei der Effektivität des gesamten Assay eine entscheidende Bedeutung zukommen und es sollte daher eine möglichst gleichbleibende Qualität und Konzentration angestrebt werden.
b) Ansatz und Durchführung der PCR mit Probanden gDNA
Für je 25 µl Gesamtvolumen/PCR Ansatz werden 2,5 µl eines 10 fach konzentrierten Polymerasepuffers (z.B. 100 mM Tris-HCl (pH 8.0), 500 mM KCI, 15 mM MgCl); 0,08 µl dNTP (62,5 mM jedes); jeweils 0,5 µl der 50 µM Primer SP2C9201 (SEQ ID NO 1) und dem 5' biotiniertem Primer RP2C9201 (SEQ ID NO 2); 0,5 µl Taq-DNA Polymerase (5U/µl), 1 µl gDNA ; sowie 16,34 µl für die PCR geeignetes Bidest im Doppelansatz in PCR Gefäße pipettiert. Anschließend werden die Gefäße in ein handelsübliches PCR Gerät überführt und nachfolgendem PCR-Regime unterworfen:

| Zyklen | Temp. | Dauer |
|---|---|---|
| 1 x | 95°C | 3 Min. |
| 35 x | 95°C | 30 Sek. |
| | 57°C | 30 Sek. |
| | 72°C | 45 Sek. |
| 1 x | 72°C | 5 Min. |
| | 4°C | Ende |

Gleichzeitig wurden Proben bestehend aus gentechnisch gewonnener Plasmid DNA (pDNA), welche allelspezifische Fragmente des Genabschnitts CYP2C9*2 (SEQ ID NO 38) und den entsprechenden Abschnitt des Wildtypgens CYP2C9*1 (SEQ ID NO 37) enthalten, als interne Kontrollen ebenfalls im Doppelansatz mitgeführt.
Die entstandenen Amplifikationsprodukte können mittels Agarosegelelektrophorese in 3,5% Agarosegelen bei 5-10 V/cm Gelbreite 1-1½ Stunden aufgetrennt und mittels DNA Marker und Ethidiumbromid Färbung unter UV Licht visualisiert und dokumentiert werden.

### Beispiel 2

Hybridisierung und allelspezifischer Nachweis der PCR Produkte aus Beispiel 1 an SA-MTP durch stringente Waschung FITC markierter Oligonukleotidsonden

| | |
|---|---|
| Lösungen TE-NaCl: | 10 mM Tris-HCl (pH 7,5); 1 mM EDTA; 100 mM NaCl |
| HP: | 6 x SSC; 0,1% SDS; 2 x Denhardtsche Lösung (DHL) |
| SDS-WP: | 0,1 x SSC; 0,1% SDS |
| TE-Lysin: | 10 mM Tris-HCl (pH 7,5); 1 mM EDTA; 100 mM NaCl; 2% Lysin (w/v) |
| TE-RSA: | 10 mM Tris-HCl (pH 7,5); 1 mM EDTA; 100 mM NaCl; 0,05% Rinderserum Albumin (w/v) |
| TE-Tween: | 10 mM Tris-HCl (pH 7,5); 1 mM EDTA; 100 mM NaCl; 0,1 % Tween (w/v) |
| TMB: | stabilisierte 3, 3', 5, 5'-Tetramethylbenzidin/H₂O₂ Lösung der Firma DRG Diagnostics (TMBlue) |

### Durchführung

Jeweils 2 × 5 µl der in Beispiel 1 entstandenen Amplifikationsprodukte werden im Doppelansatz in eine mit 45 µl TE-NaCl vorbeschickte SA-MTP pipettiert, 20 Minuten bei 37°C unter Schütteln inkubiert und dann abgesaugt. Zur Denaturierung der doppelsträngigen DNA werden je 50 µl 0,2N NaOH zugegeben, 15 Minuten bei Raumtemperatur (RT) geschüttelt und danach abgesaugt. Dann werden je 50 µl TE-NaCl zugesetzt, über 1 Min. bei RT geschüttelt und nachfolgend abgesaugt. Anschließend werden 50 µl nachfolgend bezeichneter, in HP (Hybridisierungspuffer) vorverdünnter Hybridisierungssonden (Endkonzentration - 0,5 - bzw. 1 nM) zu jeweils einem der Doppelansätze zugegeben, 20 Minuten bei 37°C hybridisiert und anschließend abgesaugt.

### Hybridisierungssonden

CYP2C9*2:
CYP2C9*1 (WT) - FITC-5'-GAGGACCGTGTTCAAGAG -3' (SEQ ID NO 17)
CYP2C9*2 (MT) - FITC-5'-TGAGGACTGTGTTCAAGAG -3' (SEQ ID NO 18)

Durch dreifach wiederholte Zugabe von 50 µl SDS-WP je Well, Inkubation bei 45°C für 11 Minuten und anschließendes Absaugen werden nicht 100% passende Hybridisierungssonden abgewaschen. Nach dem Blocken überschüssiger, unspezifischer Bindungsstellen für Anti-FITC-IgG-POD Konjugate durch 5 Minuten Inkubation in TE-Lysin bei RT werden 50 µl des Anti-FITC-IgG-POD Konjugates in einer Konzentration von 50 ng/ml in TE-RSA zugesetzt, 20 Minuten abgedunkelt bei RT geschüttelt und anschließend abgesaugt. Nach 2 facher Waschung der Wells mit je 50 µl TE-Tween werden 50 µl/well TMB Lösung zugegeben, 12 Minuten bei RT, abgedunkelt geschüttelt, unmittelbar anschließend mit 50 µl 5M H₂SO₄ versetzt und sofort danach in einem handelsüblichen MTP Reader bei 450 nm gegen die Referenzwellenlänge 620 bzw. 630 nm vermessen.

**Tabelle 2**

| Gemessene Optische Dichte bei 450 nm (OD_{450 nm}) ausgewählter CYP2C9 Allele aus pDNA bzw. gDNA (vortypisiert durch RFLP) nach Hybridisierung mit FITC-Sonden und Behandlung entsprechend Beispiel 2 | | | |
|---|---|---|---|
| Allel (Quelle) | FITC-Sonde | OD_{450 nm} | Match / Mismatch |
| CYP2C9*1 (pDNA) | WT | 2,7 | 17,5 |
| CYP2C9*2 (pDNA) | MUT | 1,9 | 10,3 |
| CYP2C9*1/*1 (gDNA) | WT | 2,7 | 16,8 |
| CYP2C9*2/*2 (gDNA) | MUT | 1,8 | 11,1 |
| CYP2C9*1/*2 (gDNA) | WT | 2,3 | 1,2 |
| CYP2C9*1/*2 (gDNA) | MUT | 1,8 | 0,8 |
| Legende: CYP2C9*1 (pDNA) ist gentechnisch hergestellte, doppelsträngige Plasmid DNA mit der enthaltenen Sequenz für CYP2C9 WT (SEQ ID NO 37) und entsprechend für CYP2C9*2 (SEQ ID NO 38); CYP2C9*2/*2 bzw. -*1/*1 oder - *1/*2 gDNA sind mittels RFLP genotypisierte, genomische DNA Proben der Allele *1 (WT), *2 (MUT) bzw. *1/*2 (heterozygot); WT = FITC markiertes Oligonukleotid mit Sequenz entsprechend Wildtypallel und MUT = FITC markiertes Oligonukleotid mit Sequenz entsprechend CYP2C9*2; OD _{450 nm} = Optische Dichte bei einer Wellenlänge von 450 nm gegen 620 nm; Match/Mismatch = der Quotient der OD ₄₅₀ ₙₘ aus 100% komplementärem zu nicht komplementärem Allel-Sonden Hybriden. | | | |

### Beispiel 3

IS-PCR von CYP2C19*3 aus pDNA mittels asymmetrischer PCR gefolgt von allelspezifischer Verlängerung FITC markierter Oligonukleotide durch Taq DNA Polymerase

Ansatz und Durchführung der PCR mit gentechnisch hergestellter pDNA:

Die pDNA enthält allespezifische Fragmente des Genabschnitts CYP2C19*3 (SEQ ID NO 44) bzw. des entsprechenden Abschnitt des Wildtypgens CYP2C19*1 (SEQ ID NO 43). Für je 25 µl Gesamtvolumen/PCR Ansatz werden 2,5 µl eines 10 fach konzentrierten Polymerasepuffers (z.B. 100 mM Tris-HCl (pH 8.0), 500 mM KCl, 15 mM MgCl); 0,2 µl dNTP (62,5 mM jedes); jeweils 0,5 µl des 5 µM Primers SP2C1932 (SEQ ID NO 7) und des 50 µM Primers RP2C9201 (SEQ ID NO 8); 0,25 µl Taq-DNA Polymerase (5U/µl), 1 µl pDNA (50 pg); sowie 20,05 µl für die PCR geeignetes Bidest im Doppelansatz in PCR Gefäße pipettiert. Anschließend werden die Gefäße in ein handelsübliches PCR Gerät überführt und nachfolgendem PCR-Regime unterworfen:

| Zyklen | Temp. | Dauer |
|---|---|---|
| 1 x | 95°C | 2 Min. |
| 35 x | 95°C | 30 Sek. |
| | 55°C | 30 Sek. |
| | 72°C | 45 Sek. |
| 1 x | 72°C | 5 Min. |
| | | Pause |

und nach Zusatz von 0,5 µl/PCR Gefäß eines der 50 µM, 5' FITC markierten, allelspezifischen Primer 2C1931AS (SEQ ID NO 56) bzw. 2C1933AS (SEQ ID NO 57) erneut

| | | |
|---|---|---|
| 1 x | 95°C | 2 Min. |
| | 59°C | 45 Sek. |
| | 72°C | 3 Min. |

einer allelspezifischen Primerverlängerung unterworfen, anschließend kurzfristig bei 4°C aufbewahrt und nachfolgend weiterverarbeitet.

### Beispiel 4

Hybridisierung und allelspezifischer Nachweis der PCR Produkte aus Beispiel 3 an Streptavidin beschichtete Glasobjekträgern (SA-Chips) durch stringente Waschung FITC markierter Primerverlängerungsprodukte

| | |
|---|---|
| Lösungen TE-NaCl: | 10 mM Tris-HCl (pH 7.5); 1 mM EDTA; 100 mM NaCl |
| 6 x SSC: | 0,09 M Na-Citrat-HCl (pH 7.0); 0,9 M NaCl |
| SDS-WP: | 0,1 x SSC; 0,1% SDS |
| TE-Lysin: | 10 mM Tris-HCl (pH 7,5); 1 mM EDTA; 100 mM NaCl; 2% Lysin (w/v) |
| TE-RSA: | 10 mM Tris-HCl (pH 7,5); 1 mM EDTA; 100 mM NaCl; 0,05% Rinderserum Albumin (w/v) |
| TE-Tween: | 10 mM Tris-HCl (pH 7,5); 1 mM EDTA; 100 mM NaCl; 0,1 % Tween (w/v) |
| TMB (p): | präzipitierende, stabilisierte 3, 3', 5, 5'-Tetramethylbenzidin/H₂O₂ Lösung der Firma Seramun |

### Durchführung

Ein mit 32-64 Reaktionsarealen (Spots) ausgestatteter SA-Chip wird mit jeweils 3 µl/Spot eines 5' biotinierten, in TE-NaCl gelösten 500 pM Oligos mit der Bezeichnung B2C193_F (SEQ ID NO 58) versehen, 15 Min. bei RT in einer Humiditätskammer inkubiert, anschließend abgesaugt mit 1 mal mit 6 x SSC bei RT gewaschen. Jeweils 0,25 µl der in Beispiel 3 entstandenen Amplifikationsprodukte werden mit 1,5 µl 12 x SSC versetzt, mit ddH₂O auf 3 µl Gesamtvolumen aufgefüllt, nach Absaugen des 6 x SSC Puffers im Dreifachansatz auf einzelne Spots pipettiert, 30 Minuten bei RT bei gesättigter Humidität hybridisiert und anschließend abgesaugt. Durch 3 fache, stringente Waschung des SA-Chips mit 20 ml SDS-WP für jeweils 5 Min. bei 46°C werden nicht verlängerte, FITC markierte Mismatch Extensionprimer abgewaschen, während korrekt gepaarte, in der Extensionreaktion verlängerte Match 5' FITC-Primer/Template Kombinationen auf dem SA-Chip verbleiben. Nach dem Blocken überschüssiger, unspezifischer Bindungsstellen für Anti-FITC-IgG-POD Konjugate durch 5 Min. Inkubation in TE-Lysin bei RT werden 2 µl eines Anti-FITC-IgG-POD Konjugates in TE-RSA (1 µg/ml) zugesetzt und abgedunkelt 20 Minuten in gesättigter Humidität bei RT inkubiert. Nach 2 facher Waschung des SA-Chips mit 5 ml TE-Tween werden 2 µl/Spot der TMB (p) Fertiglösung zugegeben und abgedunkelt in gesättigter Humidität über 15 Minuten bei RT inkubiert. Die sofort anschließende Vermessung der auftretenden Violettfärbung erfolgt in einem handelsüblichen Durchlichtscanner (Agfa Scanwise) bei einer Auflösung von 250 dpi. Die Auswertung der gescannten Bilder erfolgt mit Hilfe einer neuentwickelten, speziell auf das SA-Chip Design und das verwendete farbgebende Reagenz abgestimmten Analysesoftware und wird als auf 100% normierte Wertetabelle im Excel Format als Score Werte ausgegeben.

**Tabelle 3**

| Ergebnisse der Messung der CYP2C19*1 bzw. - *3 pDNA Allele entsprechend Beispiel 4 | | | |
|---|---|---|---|
| Allel | FITC-Sonde | Score (Mw ± Stdw, n = 3) | |
| | | 1 x PE | 2 x PE |
| CYP 2C19*1 CYP 2C 19*3 | MUT | 1 ± 1 | 2 ± 3 |
| | WT | 82 ± 13 | 85 ± 1 |
| | MUT | 70 ± 16 | 81 ± 4 |
| | WT | 6 ± 2 | 26 ± 9 |

| | | Match / Mismatch (Mw) | |
|---|---|---|---|
| | | 1 x PE | 2 x PE |
| CYP 2C19*1 | WT | 82 | 43 |
| CYP 2C 19*3 | MUT | 12 | 3 |
| Legende: CYP2C19*1 ist gentechnisch hergestellte, doppelsträngige Plasmid DNA mit der enthaltenen Sequenz für CYP2C19 WT (SEQ ID NO 43) und entsprechend für CYP2C19*3 (SEQ ID NO 44); FITC-Sonde = 5' Fluoroescein markiertes Oligonukleotid; WT = FITC markiertes Oligonukleotid mit Sequenz entsprechend Wildtypallel und MUT = FITC markiertes Oligonukleotid mit Sequenz entsprechend CYP2C19*3; Score _{(Mw ± Stdw, n = 3)} = Mittelwerte von auf 100% normierten Scorewerten ± Standardabweichung bei Dreifachansatz; Match/Mismatch _{(Mw)} = Quotient der mittleren Scorewerte aus 100% komplementärem und nicht komplementärem Allel Sonden Hybrid; n x PE = Zahl (n) der durchgeführten FITC-Primer extension Zyklen. | | | |

### Beispiel 5

Isoformspezifische Amplifikation von Genabschnitten aus CYP2C9, CYP2C19 und CYP2D6 aus genomischer Leukozyten DNA mittels Multiplex-PCR
a) Isolation der genomischen DNA (gDNA)
- siehe oben

b) Ansatz und Durchführung der PCR mit Probanden gDNA
Für je 25 µL Gesamtvolumen/PCR Ansatz werden 12,5 µL eines 2 fach konzentrierten PCR Ready Mixes (enthält: 1,25 U Taq Polymerase, Tris-HCl (pH 8.7), KCl, (NH₄)₂SO₄, 3 mM MgCl, 400 µM jeden dNTP's); jeweils 0,1 µL der Primer SP2C9201 (50 µM), RP2C9201 (50 µM), SP2C9301 (25 µM), RP2C9302 (25 µM), SP2C1921 (100 µM), RP2C1921 (100 µM), SP2C1932 (75 µM), RP2C1931 (75 µM), SP2D6322 (100 µM), RP2D6301 (100 µM), SPPUC01 (25 µM), RPPUC01 (25 µM) [DE 102 37 691.3-41 SEQ ID NO 1-10; Anhang 1 SEQ ID No 1 und 2], 2 µL gDNA, 0,5 µL pDNA pUC 19 (10 pg/µL) [SEQ ID NO 61] sowie 8,8 µL für die PCR geeignetes Bidest in PCR Gefäße pipettiert. Anschließend werden die Gefäße in ein handelsübliches PCR Gerät überführt und nachfolgendem PCR-Regime unterworfen:

| Zyklen | Temp. | Dauer |
|---|---|---|
| 1 x | 95°C | 15 Min. |
| 35 x | 95°C | 30 Sek. |
| | 57°C | 90 Sek. |
| | 72°C | 60 Sek. |
| 1 x | 72°C | 10 Min. |
| | 4°C | Ende |

Die entstandenen Amplifikationsprodukte können mittels Agarosegelelektrophorese in 3,5% Agorosegelen bei 5-10 V/cm Gelbreite 1-1½ Stunden aufgetrennt und mittels DNA Marker und Ethidiumbromid Färbung unter UV Licht visualisiert und dokumentiert werden.

### Beispiel 6

### Allelspezifische Ligation der PCR Produkte aus Beispiel 5 durch Taq DNA Ligase Detektions Reaktion im Multiplexansatz

2 µL des in Beispiel 1 entstandenen PCR Produkts werden mit 4 µL 10 x Taq Ligationspuffer (200 mM Tris-HCl (pH 7.6), 250 mM Kalium-Acetat, 100 mM Magnesium-Acetat, 100 mM Dithiothreitol, 10 mM NAD, 1 % Triton X 100), 15 U Taq DNA Ligase (New England Biolabs), je 4 pmole eines jeden Zip Code Alleloligonukleotids [SEQ ID NO 62 - 71; 76 u. 77], je 2 pmole eines jeden 5' phosphorylierten, 3' FITC markierten Ligationsoligonukleotids [SEQ ID NO 78 - 83 u. 88] versetzt und mit sterilem Bidest auf ein Gesamtvolumen von 40 µL aufgefüllt. Danach wird der Ansatz in einen handelsüblichen Thermocycler bei nachfolgendem Regime prozessiert:

| Zyklen | Temp. | Dauer |
|---|---|---|
| 1 x | 97°C | 2 Min. |
| 20 x | 97°C | 30 Sek. |
| | 65°C | 2 Min. |

### Beispiel 7

Zip Code spezifische Hybridisierung der LDR Produkte aus Beispiel 6 an vorbelegte Polycarbonatobjekträger und anschließende Detektion durch nachgeschalteten anti-FITC-Ak-HRP Elisa

40 µL Ansatz aus Beispiel 5 werden in ein Gesamtvolumen von 450 µL zu 6 x SSC, 2 x DHL, 100 µg/mL Lachs DNA Hybridisierungslösung verdünnt. Ein PC-Chip mit insgesamt 24 durch Siebdruckverfahren erzeugten Kavitäten, die mit Streptavidin vorbeschichtet wurden, wird mit jeweils 1,5 pmole aller nachfolgend über ihre Sequenz ID benannten 3' biotinierten 24 bp Oligonukleotide im Zweierreplika belegt [SEQ ID NO 89 - 98; 103 u. 104]. Diese sind jeweils revers komplimentär zu den mit jeweils einem Allel assoziierten ZIP Codes der Ligationsprodukte und gewährleisten so eine effiziente und spezifische Hybridisierung auf den PC-Chip. Der Chip wird in eine dichtende Polycarbonatkammer eingelegt, über ein Septum mit der Hybridisierungslösung überschichtet und bei 37°C 1 Stunde über Kopf rotierend inkubiert. Nach Abschluß der Hybridisierung wird der Chip mit 2 × 650 µL 1 × SSC, 0.1% SDS jeweils 15 Minuten bei 37°C rotierend gewaschen und anschließend 20 Minuten bei Raumtemperatur mit 650 µL anti-FITC-Ak-HRP (75 ng/mL) rotierend inkubiert. Nach 2 facher Waschung des SA-Chips mit 650 µL TE-Tween werden 700 µL TMB Färblösung zugegeben und 15 Minuten bei RT inkubiert. Die sofort anschließende Vermessung der auftretenden Violettfärbung erfolgt in einem handelsüblichen Durchlichtscanner (Agfa Scanwise) bei einer Auflösung von 250 dpi. Die Auswertung der gescannten Bilder erfolgt mit Hilfe einer neuentwickelten, speziell auf das SA-Chip Design und das verwendete farbgebende Reagenz abgestimmten Analysesoftware und wird als auf 100% normierte Wertetabelle im Excel Format als Score Werte ausgegeben.

**Tabelle 4**

| Ergebnisse der Messung einer gDNA Probe entsprechend Beispiel 7 | | | | |
|---|---|---|---|---|
| Isoform | | Score _{(Mw ± Stdw, n = 3)} | S/N | Genotyp |
| CYP2C9 | *1 | 91 ± 2 | 10 | *1 |
| | *2 | 9 ± 1 | | |
| CYP2C9 | *1 | 91 ± 1 | 6,5 | |
| | *3 | 14 ± 1 | | |
| CYP2C19 | *1 | 89 ± 1 | 22,5 | *1 |
| | *2 | 4 ± 5 | | |
| CYP2C19 | *1 | 97 ± 2 | 12 | |
| | *3 | 8 ± 2 | | |
| CYP2D6 | *1 | 1 ± 1 | 81 | *3 |
| | *3 | 81 ± 6 | | |
| pUC | Pos. | 83 ± 3 | 21 | |
| | Neg. | 4 ± 3 | | |
| Legende: S/N = signal noise ratio (Signal/Rausch Verhältnis); Mw = Mittelwert; Stdw = Standardabweichung; Pos. = Postivkontrolle; Neg. = Negativkontrolle | | | | |

Anlage 1: (Sequenzprotokoll)

Künstliche Oligonukleotide und Plasmid DNA Teilsequenzen

<210> 1
<211> 23
<212> DNA
<213> künstliches Oligonukleotid

<220>
<223> Primer: SP2C9201

<400> 1
cgtttctctt cctgttagga att 23

<210> 2
<211> 20
<212> DNA
<213> künstliches Oligonukleotid

<220>
<223> Primer: RP2C9201

<400> 2
ggtcagtgat atggagtagg 20

<210> 3
<211> 22
<212> DNA
<213> künstliches Oligonukleotid

<220>
<223> Primer: SP2C9301

<400> 3
ccaggaagag attgaacgtg tg 22

<210> 4
<211> 22
<212> DNA
<213> künstliches Oligonukleotid

<220>
<223> Primer: RP2C9302

<400> 4
acatggagtt gcagtgtagg ag 22

<210> 5
<211> 18
<212> DNA
<213> künstliches Oligonukleotid

<220>
<223> Primer: SP2C1921

<400> 5
aattacaacc agagcttggc                                                    20


<210> 6
<211> 18
<212> DNA
<213> künstliches Oligonukleotid

<220>
<223> Primer: RP2C1921

<400> 6
gggttgttgatgtccatc                                                       18


<210> 7
<211> 20
<212> DNA
<213> künstliches Oligonukleotid

<220>
<223> Primer: SP2C1932

<400> 7
gctccctgca atgtgatctg                                                    20


<210> 8
<211> 22
<212> DNA
<213> künstliches Oligonukleotid

<220>
<223> Primer: RP2C1931

<400> 8
ggatttccca gaaaaaaga ct                                                  22


<210> 9
<211> 23

<212> DNA
<213> künstliches Oligonukleotid

<220>
<223> Primer: SP2D6302

<400> 9
atgctgtccc cgtcctcctg cat                                              23


<210> 10
<211> 18
<212> DNA
<213> künstliches Oligonukleotid

<220>
<223> Primer: RP2D6301

<400> 10
tggtcaccat cccggcag                                                    18


<210> 11
<211> 20
<212> DNA
<213> künstliches Oligonukleotid

<220>
<223> Primer: SP2D6404

<400> 11
aatgccttca tggccacgcg                                                  20


<210> 12
<211> 19
<212> DNA
<213> künstliches Oligonukleotid

<220>
<223> Primer: RP2D6401

<400> 12
agctcgccct gcagagact                                                   19


<210> 13
<211> 20
<212> DNA
<213> künstliches Oligonukleotid

<220>
<223> Primer: SP2C801

<400> 13
cagggcttgg tgtaagatac                                                    20


<210> 14
<211> 21
<212> DNA
<213> künstliches Oligonukleotid

<220>
<223> Primer: RP2C802

<400> 14
ggcctgatca tttaaacatc c                                                  21


<210> 15
<211> 23
<212> DNA
<213> künstliches Oligonukleotid

<220>
<223> Primer: SP2C807

<400> 15
ccataatggc attactgact tcc                                                23


<210> 16
<211> 24
<212> DNA
<213> künstliches Oligonukleotid

<220>
<223> Primer: RP2C806

<400> 16
agtcactttt cttaaagttg ccat                                               24


<210> 17
<211> 18
<212> DNA
<213> künstliches Oligonukleotid

<220>
<223> Sonde: H2C92103

<400> 17
gaggaccgtg ttcaagag                                                    18


<210> 18
<211> 19
<212> DNA
<213> künstliches Oligonukleotid

<220>
<223> Sonde: H2C92204

<400> 18
tgaggactgt gttcaagag                                                   19


<210> 19
<211> 20
<212> DNA
<213> künstliches Oligonukleotid

<220>
<223> Sonde: HYB2C931

<400> 19
ccagagatac attgaccttc                                                  20


<210> 20
<211> 20
<212> DNA
<213> künstliches Oligonukleotid

<220>
<223> Sonde: HYB2C933

<400> 20
ccagagatac cttgaccttc                                                  20


<210> 21
<211> 19
<212> DNA
<213> künstliches Oligonukleotid

<220>
<223> Sonde: HB2C1921

<400> 21
gattatttcc cgggaaccc                                                   19

<210> 22
<211> 22
<212> DNA
<213> künstliches Oligonukleotid

<220>
<223> Sonde: HB2C19228

<400> 22
gattatttcc caggaaccca ta                                                22


<210> 23
<211> 16
<212> DNA
<213> künstliches Oligonukleotid

<220>
<223> Sonde: HB2C19312

<400> 23
   caccccctgg atccag                                                      16


<210> 24
<211> 16
<212> DNA
<213> künstliches Oligonukleotid

<220>
<223> Sonde: HB2C19332

<400> 24
caccccctga atccag                                                       16


<210> 25
<211> 19
<212> DNA
<213> künstliches Oligonukleotid

<220>
<223> Sonde: HY2D6312

<400> 25
aactgagcac aggatgacc                                                    19


<210> 26
<211> 18

<212> DNA
<213> künstliches Oligonukleotid

<220>
<223> Sonde: HY2D6331

<400> 26
actgagcacg gatgacct                                                    18


<210> 27
<211> 20
<212> DNA
<213> künstliches Oligonukleotid

<220>
<223> Sonde: HY2D64110

<400> 27
acccccagga igcccctttc                                                  20


<210> 28
<211> 18
<212> DNA
<213> künstliches Oligonukleotid

<220>
<223> Sonde: HY2D6441

<400> 28
acccccaaga cgcccctt                                                    18


<210> 29
<211> 17
<212> DNA
<213> künstliches Oligonukleotid

<220>
<223> Sonde: HY2D6614

<400> 29
gagcagtggg tgaccga                                                     17


<210> 30
<211> 16
<212> DNA
<213> künstliches Oligonukleotid

<220>
<223> Sonde: HY2D6664

<400> 30
gagcaggggt gaccga                                                    16

<210> 31
<211> 19
<212> DNA
<213> künstliches Oligonukleotid

<220>
<223> Sonde: HY2C8212

<400> 31
tgcttcctga tcaaaatgg                                                 19

<210> 32
<211> 19
<212> DNA
<213> künstliches Oligonukleotid

<220>
<223> Sonde: HY2C8222

<400> 32
tgcttcctgt tcaaaatgg                                                 19

<210> 33
<211> 19
<212> DNA
<213> künstliches Oligonukleotid

<220>
<223> Sonde: HY2C8412

<400> 33
gggactttat cgattgctt                                                 19

<210> 34
<211> 19
<212> DNA
<213> künstliches Oligonukleotid

<220>
<223> Sonde: HY2C8442

<400> 34
gggactttat ggattgctt                                                    19


<210> 35
<211> 23
<212> DNA
<213> künstliches Oligonukleotid


<220>
<223> Sonde: HY2C8314

<400> 35
tacatgatga caaagaattt cct                                               23


<210> 36
<211> 23
<212> DNA
<213> künstliches Oligonukleotid

<220>
<223> Sonde: HY2C8334

<400> 36
tacatgatga cagagaattt cct                                               23


<210> 37
<211> 197
<212> DNA
<213> Plasmid DNA (sequenzspezifischer Teil)

<220>
<223> pDNA CYP2C9*2 WT

<400> 37
cgtttctctt cctgttagga attgttttca gcaatggaaa gaaatggaag      50
gagatccggc gtttctccct catgacgctg cggaatttgg ggatggggaa      100
gaggagcatt gaggaccgtg ttcaagagga agcccgctgc cttgtggagg      150
agttgagaaa aaccaagggt gggtgaccct actccatatc actgacc         197


<210> 38
<211> 197
<212> DNA
<213> Plasmid DNA (sequenzspezifischer Teil)

<220>

<223> pDNA CYP2C9*2 MUT

<400> 38
cgtttctctt cctgttagga attgttttca gcaatggaaa gaaatggaag 50
gagatccggc gtttctccct catgacgctg cggaatttgg ggatggggaa 100
gaggagcatt gaggactgtg ttcaagagga agcccgctgc cttgtggagg 150
agttgagaaa aaccaagggt gggtgaccct actccatatc actgacc 197

<210> 39
<211> 215
<212> DNA
<213> Plasmid DNA (sequenzspezifischer Teil)

<220>
<223> pDNA CYP2C9*3 WT

<400> 39
ccaggaagag attgaacgtg tgattggcag aaaccggagc ccctgcatgc 50
aagacaggag ccacatgccc tacacagatg ctgtggtgca cgaggtccag 100
agatacattg accttctccc caccagcctg ccccatgcag tgacctgtga 150
cattaaattc agaaactatc tcattcccaa ggtaagtttg tttctcctac 200
actgcaactc catgt 215

<210> 40
<211> 215
<212> DNA
<213> Plasmid DNA (sequenzspezifischer Teil)

<220>
<223> pDNA CYP2C9*3 MUT

<400> 40
ccaggaagag attgaacgtg tgattggcag aaaccggagc ccctgcatgc 50
aagacaggag ccacatgccc tacacagatg ctgtggtgca cgaggtccag 100
agataccttg accttctccc caccagcctg ccccatgcag tgacctgtga 150
cattaaattc agaaactatc tcattcccaa ggtaagtttg tttctcctac 200
actgcaactc catgt 215

<210> 41
<211> 216
<212> DNA
<213> Plasmid DNA (sequenzspezifischer Teil)

<220>
<223> pDNA CYP2C19*2 WT

<400> 41
aattacaacc agagcttggc atattgtatc tataccttta ttaaatgctt 50
ttaatttaat aaattattgt tttctcttag atatgcaata attttcccac 100

tatcattgat tatttcccgg gaacccataa caaattactt aaaaaccttg 150
cttttatgga aagtgatatt ttgagaaagt aaaagaacac caagaatcga 200
tggacatcaa caaccc 216

<210> 42
<211> 216
<212> DNA
<213> Plasmid DNA (sequenzspezifischer Teil)

<220>
<223> pDNA CYP2C19*2 MUT

<400> 42
aattacaacc agagcttggc atattgtatc tataccttta ttaaatgctt 50
ttaatttaat aaattattgt tttctcttag atatgcaata attttcccac 100
tatcattgat tatttcccag gaacccataa caaattactt aaaaaccttg 150
cttttatgga aagtgatatt ttgagaaagt aaaagaacac caagaatcga 200
tggacatcaa caaccc 216

<210> 43
<211> 215
<212> DNA
<213> Plasmid DNA (sequenzspezifischer Teil)

<220>
<223> pDNA CYP2C19*3 WT

<400> 43
gctccctgca atgtgatctg ctccattatt ttccagaaac gtttcgatta 50
taaagatcag caatttctta acttgatgga aaaattgaat gaaaacatca 100
ggattgtaag caccccctgg atccaggtaa ggccaagttt tttgcttcct 150
gagaaaccac ttacagtctt tttttctggg aaatccaaaa ttctatattg 200
accaagccct gaagt 215

<210> 44
<211> 215
<212> DNA
<213> Plasmid DNA (sequenzspezifischer Teil)

<220>
<223> pDNA CYP2C19*3 MUT

<400> 44
gctccctgca atgtgatctg ctccattatt ttccagaaac gtttcgatta 50
taaagatcag caatttctta acttgatgga aaaattgaat gaaaacatca 100
ggattgtaag caccccctga atccaggtaa ggccaagttt tttgcttcct 150
gagaaaccac ttacagtctt tttttctggg aaatccaaaa ttctatattg 200
accaagccct gaagt 215

<210> 45
<211> 663
<212> DNA
<213> Plasmid DNA (sequenzspezifischer Teil)

<220>
<223> pDNA CYP2D6*3 WT

<400> 45
tgtacctcct atccacgtca gagatttcga ttttaggttt ctcctctggg 50
caaggagaga gggtggaggc tggcacttgg ggagggactt ggtgaggtca 100
gtggtaagga caggcaggcc ctgggtctac ctggagatgg ctggggcctg 150
agacttgtcc aggtgaacgc agagcacagg agggattgag accccgttct 200
gtctggtgta ggtgctgaat gctgtccccg tcctcctgca tatcccagcg 250
ctggctggca aggtcctacg cttccaaaag gctttcctga cccagctgga 300
tgagctgcta actgagcaca ggatgacctg ggacccagcc cagccccccc 350
gagacctgac tgaggccttc ctggcagaga tggagaaggt gagagtggct 400
gccacggtgg ggggcaaggg tggtgggttg agcgtcccag gaggaatgag 450
gggaggctgg gcaaaaggtt ggaccagtgc atcacccggc gagccgcatc 500
tgggctgaca ggtgcagaat tggaggtcat ttgggggcta ccccgttctg 550
tcccgagtat gctctcggcc ctgctcaggc caaggggaac cctgagagca 600
gcttcaatga tgagaacctg cgcatagtgg tggctgacct gttctctgcc 650
gggatggtga cca 663

<210> 46
<211> 663
<212> DNA
<213> Plasmid DNA (sequenzspezifischer Teil)

<220>
<223> pDNA CYP2D6*3 MUT

<400> 46
tgtacctcct atccacgtca gagatttcga ttttaggttt ctcctctggg 50
caaggagaga gggtggaggc tggcacttgg ggagggactt ggtgaggtca 100
gtggtaagga caggcaggcc ctgggtctac ctggagatgg ctggggcctg 150
agacttgtcc aggtgaacgc agagcacagg agggattgag accccgttct 200
gtctggtgta ggtgctgaat gctgtccccg tcctcctgca tatcccagcg 250
ctggctggca aggtcctacg cttccaaaag gctttcctga cccagctgga 300
tgagctgcta actgagcacg gatgacctgg gacccagccc agcccccccg 350
agacctgact gaggccttcc tggcagagat ggagaaggtg agagtggctg 400
ccacggtggg gggcaagggt ggtgggttga gcgtcccagg aggaatgagg 450
ggaggctggg caaaaggttg gaccagtgca tcacccggcg agccgcatct 500
gggctgacag gtgcagaatt ggaggtcatt tgggggctac cccgttctgt 550
cccgagtatg ctctcggccc tgctcaggcc aaggggaacc ctgagagcag 600
cttcaatgat gagaacctgc gcatagtggt ggctgacctg ttctctgccg 650
ggatggtgac ca 662

<210> 47
<211> 652
<212> DNA
<213> Plasmid DNA (sequenzspezifischer Teil)

<220>
<223> pDNA CYP2D6*4 WT

<400> 47
tagggttgga gtgggtggtg gatggtgggg ctaatgcctt catggccacg 50
cgcacgtgcc cgtcccaccc ccaggggtgt tcctggcgcg ctatgggccc 100
gcgtggcgcg agcagaggcg cttctccgtg tccaccttgc gcaacttggg 150
cctgggcaag aagtcgctgg agcagtgggt gaccgaggag gccgcctgcc 200
tttgtgccgc cttcgccaac cactccggtg ggtgatgggc agaagggcac 250
aaagcgggaa ctgggaaggc gggggacggg gaaggcgacc ccttacccgc 300
atctcccacc cccaggacgc ccctttcgcc ccaacggtct cttggacaaa 350
gccgtgagca acgtgatcgc ctccctcacc tgcgggcgcc gcttcgagta 400
cgacgaccct cgcttcctca ggctgctgga cctagctcag gagggactga 450
aggaggagtc gggctttctg cgcgaggtgc ggagcgagag accgaggagt 500
ctctgcaggg cgagctcccg agaggtgccg gggctggact ggggcctcgg 550
aagagcagga tttgcataga tgggtttggg aaaggacatt ccaggagacc 600
ccactgtaag aagggcctgg aggaggaggg gacatctcag acatggtcgt 650
gg 652

<210> 48
<211> 652
<212> DNA
<213> Plasmid DNA (sequenzspezifischer Teil)

<220>
<223> pDNA CYP2D6*4 MUT

<400> 48
tagggttgga gtgggtggtg gatggtgggg ctaatgcctt catggccacg 50
cgcacgtgcc cgtcccaccc ccaggggtgt tcctggcgcg ctatgggccc 100
gcgtggcgcg agcagaggcg cttctccgtg tccaccttgc gcaacttggg 150
cctgggcaag aagtcgctgg agcagtgggt gaccgaggag gccgcctgcc 200
tttgtgccgc cttcgccaac cactccggtg ggtgatgggc agaagggcac 250
aaagcgggaa ctgggaaggc gggggacggg gaaggcgacc ccttacccgc 300
atctcccacc cccaagacgc ccctttcgcc ccaacggtct cttggacaaa 350
gccgtgagca acgtgatcgc ctccctcacc tgcgggcgcc gcttcgagta 400
cgacgaccct cgcttcctca ggctgctgga cctagctcag gagggactga 450
aggaggagtc gggctttctg cgcgaggtgc ggagcgagag accgaggagt 500
ctctgcaggg cgagctcccg agaggtgccg gggctggact ggggcctcgg 550
aagagcagga tttgcataga tgggtttggg aaaggacatt ccaggagacc 600
ccactgtaag aagggcctgg aggaggaggg gacatctcag acatggtcgt 650
gg 652

<210> 49
<211> 484
<212> DNA
<213> Plasmid DNA (sequenzspezifischer Teil)

<220>
<223> pDNA CYP2D6*6 WT

<400> 49
aatgccttca tggccacgcg cacgtgcccg tcccaccccc aggggtgttc 50
ctggcgcgct atgggcccgc gtggcgcgag cagaggcgct tctccgtgtc 100
caccttgcgc aacttgggcc tgggcaagaa gtcgctggag cagtgggtga 150
ccgaggaggc cgcctgcctt tgtgccgcct tcgccaacca ctccggtggg 200
tgatgggcag aagggcacaa agcgggaact gggaaggcgg gggacgggga 250
aggcgacccc ttacccgcat ctcccacccc caggacgccc ctttcgcccc 300
aacggtctct tggacaaagc cgtgagcaac gtgatcgcct ccctcacctg 350
cgggcgccgc ttcgagtacg acgaccctcg cttcctcagg ctgctggacc 400
tagctcagga gggactgaag gaggagtcgg gctttctgcg cgaggtgcgg 450
agcgagagac cgaggagtct ctgcagggcg agct 484

<210> 50
<211> 484
<212> DNA
<213> Plasmid DNA (sequenzspezifischer Teil)

<220>
<223> pDNA CYP2D6*6 MUT

<400> 50
aatgccttca tggccacgcg cacgtgcccg tcccaccccc aggggtgttc 50
ctggcgcgct atgggcccgc gtggcgcgag cagaggcgct tctccgtgtc 100
caccttgcgc aacttgggcc tgggcaagaa gtcgctggag caggggtgac 150
cgaggaggcc gcctgccttt gtgccgcctt cgccaaccac tccggtgggt 200
gatgggcaga agggcacaaa gcgggaactg ggaaggcggg ggacggggaa 250
ggcgacccct tacccgcatc tcccaccccc aggacgcccc tttcgcccca 300
acggtctctt ggacaaagcc gtgagcaacg tgatcgcctc cctcacctgc 350
gggcgccgct tcgagtacga cgaccctcgc ttcctcaggc tgctggacct 400
agctcaggag ggactgaagg aggagtcggg ctttctgcgc gaggtgcgga 450
gcgagagacc gaggagtctc tgcagggcga gct 483

<210> 51
<211> 342
<212> DNA
<213> Plasmid DNA (sequenzspezifischer Teil)

<220>
<223> pDNA CYP2C8*2*4 WT

<400> 51

cagggcttgg tgtaagatac atatatctta tgacatgttt atatttaata 50
ttcttttctc ttttaggtct gcaataattt ccctctactc attgattgtt 100
tcccaggaac tcacaacaaa gtgcttaaaa atgttgctct tacacgaagt 150
tacattaggg agaaagtaaa agaacaccaa gcatcactgg atgttaacaa 200
tcctcgggac tttatcgatt gcttcctgat caaaatggag caggtaagat 250
attagcaaca gatcagtatt ttgatttctt gtccattttg tgattcatcg 300
aatccttctg taatttacta aggatgttta aatgatcagg cc 342

<210> 52
<211> 342
<212> DNA
<213> Plasmid DNA (sequenzspezifischer Teil)

<220>
<223> pDNA CYP2C8*2 MUT

<400> 52
cagggcttgg tgtaagatac atatatctta tgacatgttt atatttaata 50
ttcttttctc ttttaggtct gcaataattt ccctctactc attgattgtt 100
tcccaggaac tcacaacaaa gtgcttaaaa atgttgctct tacacgaagt 150
tacattaggg agaaagtaaa agaacaccta gcatcactgg atgttaacaa 200
tcctcgggac tttatcgatt gcttcctgat caaaatggag caggtaagat 250
attagcaaca gatcagtatt ttgatttctt gtccattttg tgattcatcg 300
aatccttctg taatttacta aggatgttta aatgatcagg cc 342

<210> 53
<211> 342
<212> DNA
<213> Plasmid DNA (sequenzspezifischer Teil)

<220>
<223> pDNA CYP2C8*4 MUT

<400> 53
cagggcttgg tgtaagatac atatatctta tgacatgttt atatttaata 50
ttcttttctc ttttaggtct gcaataattt ccctctactc attgattgtt 100
tcccaggaac tcacaacaaa gtgcttaaaa atgttgctct tacacgaagt 150
tacattaggg agaaagtaaa agaacaccaa gcatcactgg atgttaacaa 200
tcctcgggac tttatggatt gcttcctgat caaaatggag caggtaagat 250
attagcaaca gatcagtatt ttgatttctt gtccattttg tgattcatcg 300
aatccttctg taatttacta aggatgttta aatgatcagg cc 342

<210> 54
<211> 114
<212> DNA
<213> Plasmid DNA (sequenzspezifischer Teil)

<220>

<223> pDNA CYP2C8*3 WT

<400> 54
ccataatggc attactgact tccgtgctac atgatgacaa agaatttcct 50
aatccaaata tctttgaccc tggccacttt ctagataaga atggcaactt 100
taagaaaagt gact 114

<210> 55
<211> 114
<212> DNA
<213> Plasmid DNA (sequenzspezifischer Teil)

<220>
<223> pDNA CYP2C8*3 MUT

<400> 55
ccataatggc attactgact tccgtgctac atgatgacag agaatttcct 50
aatccaaata tctttgaccc tggccacttt ctagataaga atggcaactt 100
taagaaaagt gact 114

<210> 56
<211> 20
<212> DNA
<213> künstliches Oligonukleotid

<220>
<223> Primer: 2C1931AS

<400> 56
   ggattgtaag caccccctgg 20

<210> 57
<211> 20
<212> DNA
<213> künstliches Oligonukleotid

<220>
<223> Primer: 2C1933AS

<400> 57
   ggattgtaag caccccctga 20

<210> 58
<211> 34
<212> DNA
<213> künstliches Oligonukleotid

<220>
<223> Primer: B2C193_F

<400> 58
gctccctgca atgtgatctg ctccattatt ttcc                                34

Anlage 2: (Sequenzprotokoll)

Künstliche Oligonukleotide und Plasmid DNA Teilsequenzen

<210> 59
<211> 20
<212> DNA
<213> künstliches Oligonukleotid
<220>
<223> Primer: SPpUC01

<400> 59
CCTCTGACAC ATGCAGCTCC 20

<210> 60
<211> 20
<212> DNA
<213> künstliches Oligonukleotid
<220>
<223> Primer: RPpUC01

<400> 60
AATCGCCTTG CAGCACATCC 20

<210> 61
<211> 312
<212> DNA
<213> E. coli
<220>
<223> pUC Plasmid (partial)

<400> 61
cctctgacac atgcagctcc cggagacggt cacagcttgt ctgtaagcgg 50
atgccgggag cagacaagcc cgtcagggcg cgtcagcggg tgttggcggg 100
tgtcggggct ggcttaacta tgcggcatca gagcagattg tactgagagt 150

gcaccatatg cggtgtgaaa taccgcacag atgcgtaagg agaaaatacc 200
gcatcaggcg ccattcgcca ttcaggctgc gcaactgttg ggaagggcga 250
tcggtgcggg cctcttcgct attacgccag ctggcgaaag ggggatgtgc 300
tgcaaggcga tt 312

<210> 62

<211> 45

<212> DNA

<213> künstliches Oligonukleotid

<220>

<223> LDR Oligo: Z2C92WT

<400> 62

ctcgtggaac gcaagcgcat gtcgggaaga ggagcattga ggacc 45

<210> 63

<211> 45

<212> DNA

<213> künstliches Oligonukleotid

<220>

<223> LDR Oligo: Z2C92MT

<400> 63

cagcacctga ccatcgatcg cagcggaaga ggagcattga ggact 45

<210> 64

<211> 45

<212> DNA

<213> künstliches Oligonukleotid

<220>

<223> LDR Oligo: Z2C93WT

<400> 64

gagcaccttg cgaacgcgta cagctgcacg aggtccagag ataca 45

<210> 65

<211> 45

<212> DNA

<213> künstliches Oligonukleotid

<220>

<223> LDR Oligo: Z2C93MT

<400> 65

gtcggcataa gctgcgctca acggtgcacg aggtccagag atacc 45

<210> 66

<211> 47

<212> DNA

<213> künstliches Oligonukleotid

<220>

<223> LDR Oligo: Z2C192WT

<400> 66

gtcctccttg cgatcgggta cagcccacta tcattgatta tttcccg 47

<210> 67

<211> 47

<212> DNA

<213> künstliches Oligonukleotid

<220>

<223> LDR Oligo: Z2C192MT

<400> 67

tcgcggaaca ccacctacct tgcgccacta tcattgatta tttccca 47

<210> 68

<211> 44

<212> DNA

<213> künstliches Oligonukleotid

<220>

<223> LDR Oligo: Z2C193WT

<400> 68

ctggaggaac gctagcccat gtcgggattg taagcacccc ctgg 44

<210> 69

<211> 44

<212> DNA

<213> künstliches Oligonukleotid

<220>

<223> LDR Oligo: Z2C193MT

<400> 69

acgccctagt cgtggatgga acgcggattg taagcacccc ctga 44

<210> 70

<211> 47

<212> DNA

<213> künstliches Oligonukleotid

<220>

<223> LDR Oligo: Z2D63WT

<400> 70

cagccgaaga ccacctatcg tgcgggatga gcagctaact gagcaca 47

<210> 71

<211> 47

<212> DNA

<213> künstliches Oligonukleotid

<220>

<223> LDR Oligo: Z2D63MT

<400> 71
gtgcgctatg cgacctggta tgcgggatga gcagctaact gagcacg        47

<210> 72
<211> 41
<212> DNA
<213> künstliches Oligonukleotid
<220>
<223> LDR Oligo: Z2D64WT

<400> 72
gtcggcatct ggtggatagc aggcgggcga aaggggcgtc c              41

<210> 73
<211> 41
<212> DNA
<213> künstliches Oligonukleotid
<220>
<223> LDR Oligo: Z2D64MT

<400> 73
ctgcccttac gctggaccat acgcgcatct cccaccccca a              41

<210> 74
<211> 44
<212> DNA
<213> künstliches Oligonukleotid
<220>
<223> LDR Oligo: Z2D66WT

<400> 74
gtcgtagcct cgccattagc cacgcaagaa gtcgctggag cagt           44

<210> 75

<211> 44

<212> DNA

<213> künstliches Oligonukleotid

<220>

<223> LDR Oligo: Z2D66MT

<400> 75

gaggttcgtg cgcgtaggta gagggcggcc tcctcggtca cccc 44

<210> 76

<211> 44

<212> DNA

<213> künstliches Oligonukleotid

<220>

<223> LDR Oligo: ZpUCWT

<400> 76

atggagcacg agtcgttacg cacctggctt aactatgcgg catc 44

<210> 77

<211> 44

<212> DNA

<213> künstliches Oligonukleotid

<220>

<223> LDR Oligo: ZpUCMT

<400> 77

ctgaggatcg aatcgccatg cttgtggctt aactatgcgg catt 44

<210> 78

<211> 20

<212> DNA

<213> künstliches Oligonukleotid

<220>

<223> LDR Oligo: Lig2C9_2

<400> 78
gtgttcaaga ggaagcccgc                                                    20

<210> 79
<211> 20
<212> DNA
<213> künstliches Oligonukleotid
<220>
<223> LDR Oligo: Lig2C9_3

<400> 79
ttgaccttct ccccaccagc                                                    20

<210> 80
<211> 27
<212> DNA
<213> künstliches Oligonukleotid
<220>
<223> LDR Oligo: Lig2C19_2

<400> 80
ggaac ccata acaaattact taaaaac                                           27

<210> 81
<211> 18
<212> DNA
<213> künstliches Oligonukleotid
<220>
<223> LDR Oligo: Lig2C19_3

<400> 81
atccaggtaa ggccaagt                                                      18

<210> 82
<211> 19
<212> DNA
<213> künstliches Oligonukleotid
<220>
<223> LDR Oligo: Lig2D6_3W

<400> 82
ggatgacctg ggacgcagc                                                    19

<210> 83
<211> 19
<212> DNA
<213> künstliches Oligonukleotid
<220>
<223> LDR Oligo: Lig2D6_3M

<400> 83
gatgacctgg gacgcagcc                                                    19

<210> 84
<211> 16
<212> DNA
<213> künstliches Oligonukleotid
<220>
<223> LDR Oligo: Lig2D6_4W

<400> 84
tgggggtggg agatgc                                                       16

<210> 85

<211> 16
<212> DNA
<213> künstliches Oligonukleotid
<220>
<223> LDR Oligo: Lig2D6_4W

<400> 85
gacgcccctt tcgccc                                                16

<210> 86
<211> 18
<212> DNA
<213> künstliches Oligonukleotid
<220>
<223> LDR Oligo: Lig2D6_6W

<400> 86
gggtgaccga ggaggccg                                              18

<210> 87
<211> 19
<212> DNA
<213> künstliches Oligonukleotid
<220>
<223> LDR Oligo: Lig2D6_6M

<400> 87
ctgctccagc gacttcttg                                             19

<210> 88
<211> 20

<212> DNA
<213> künstliches Oligonukleotid
<220>
<223> LDR Oligo: Lig_pUC

<400> 88
agagcagatt gtactgagag                                                    20

<210> 89
<211> 24
<212> DNA
<213> künstliches Oligonukleotid
<220>
<223> Hybridsierungsoligo: F2C92WT

<400> 89
cgacatgcgc ttgcgttcca cgag                                               24

<210> 90
<211> 24
<212> DNA
<213> künstliches Oligonukleotid
<220>
<223> Hybridsierungsoligo: F2C92MT

<400> 90
gctgcgatcg atggtcaggt gctg                                               24

<210> 91
<211> 24
<212> DNA

<213> künstliches Oligonukleotid
<220>
<223> Hybridsierungsoligo: F2C93WT

<400> 91
gctgtacgcg ttcgcaaggt gctc                                              24

<210> 92
<211> 24
<212> DNA
<213> künstliches Oligonukleotid
<220>
<223> Hybridsierungsoligo: F2C93MT

<400> 92
ccgttgagcg cagcttatgc cgac                                              24

<210> 93
<211> 24
<212> DNA
<213> künstliches Oligonukleotid
<220>
<223> Hybridsierungsoligo: F2C192WT

<400> 93
gctgtacccg atcgcaagga ggac                                              24

<210> 94
<211> 24
<212> DNA
<213> künstliches Oligonukleotid
<220>

<223> Hybridsierungsoligo: F2C192MT

<400> 94
cgcaaggtag gtggtgttcc gcga                                              24

<210> 95
<211> 24
<212> DNA
<213> künstliches Oligonukleotid
<220>
<223> Hybridsierungsoligo: F2C193WT

<400> 95
cgacatgggc tagcgttcct ccag                                              24

<210> 96
<211> 24
<212> DNA
<213> künstliches Oligonukleotid
<220>
<223> Hybridsierungsoligo: F2C193MT

<400> 96
gcgttccatc cacgactagg gcgt                                              24

<210> 97
<211> 24
<212> DNA
<213> künstliches Oligonukleotid
<220>
<223> Hybridsierungsoligo: F2D63WT

<400> 97
cgcacgatag gtggtcttcg gctg                                              24

<210> 98
<211> 24
<212> DNA
<213> künstliches Oligonukleotid
<220>
<223> Hybridsierungsoligo: F2D63MT

<400> 98
cgcataccag gtcgcatagc gcac                                              24

<210> 99
<211> 24
<212> DNA
<213> künstliches Oligonukleotid
<220>
<223> Hybridsierungsoligo: F2D64WT

<400> 99
gcctgctatc caccagatgc cgac                                              24

<210> 100
<211> 24
<212> DNA
<213> künstliches Oligonukleotid
<220>
<223> Hybridsierungsoligo: F2D64MT

<400> 100
gcgtatggtc cagcgtaagg gcag                                              24

<210> 101
<211> 24
<212> DNA
<213> künstliches Oligonukleotid
<220>
<223> Hybridsierungsoligo: F2D66WT

<400> 101
cgtggctaat ggcgaggcta cgac                                              24

<210> 102
<211> 24
<212> DNA
<213> künstliches Oligonukleotid
<220>
<223> Hybridsierungsoligo: F2D66MT

<400> 102
cctctaccta cgcgcacgaa cctc                                              24

<210> 103
<211> 24
<212> DNA
<213> künstliches Oligonukleotid
<220>
<223> Hybridsierungsoligo: FpUCWT

<400> 103
ggtgcgtaac gactcgtgct ccat                                              24

<210> 104
<211> 24
<212> DNA

<213> künstliches Oligonukleotid

<220>

<223> Hybridsierungsoligo: FpUCMT

<400> 104

caagcatggc gattcgatcc tcag 24

## Patentansprüche

1. Verfahren zum Nachweis von Einzelnukleotidpolymorphismen (SNP) in humanen CYP2-Genen unter Verwendung der Primer und/oder Sonden gemäß Anlage 1 und Anlage 2, wobei bevorzugt mit den beschriebenen Primern eine hochselektive Amplifikation der betreffenden CYP2-Allele erfolgt.

2. Verfahren nach Anspruch 1, **dadurch gekennzeichnet, daß** mit den beschriebenen Primern und/oder Sonden ein Nachweis der betreffenden CYP2-Allele zu Kontrollzwecken in artifiziellen Plasmiden, vorzugsweise Plasmiden gemäß Anlage 1 und Anlage 2, erfolgt.

3. Verfahren nach Anspruch 1 oder 2, **dadurch gekennzeichnet, daß** mit den beschriebenen Primern und/oder Sonden in einem Hybridisierungsassay ein Nachweis der betreffenden CYP2-Allele in genomischer DNA erfolgt, wobei sowohl homozygote als auch heterozygote Träger des betreffenden Allels nachgewiesen werden und der Hybridisierungsassay bevorzugt auf Streptavidin beschichteten Mikrotiterplatten bzw. auf Streptavidin beschichteten Glasobjekträgern erfolgt.

4. Verfahren nach Anspruch 1-3, **gekennzeichnet durch** folgende Verfahrensschritte:
a) Markierung eines Primers pro Genabschnitt mit Biotin;
b) Amplifikation allelbestimmender Genabschnitte mit Oligonukleotidprimern gemäß Anlage 1 mittels diskriminierender PCR;
c) Kopplung der markierten Amplikons an thermostabile Streptavidin-Mikrotiterplatten;
d) Entfernung der verunreinigenden genomischen DNA sowie der Komplementärstränge **durch** stringentes Waschen;
e) Hybridisierung der einzelsträngig vorliegenden gebundenen Amplikons mit allelspezifischen, FITC markierten Oligonukleotiden;
f) Entfernung der nicht gebundenen FITC markierten Oligonukleotide **durch** Waschen;
g) Detektion der allelrepräsentierenden FITC markierten Oligonukleotide unter Verwendung eines Anti-FITC-Ak-HRP ELISAs.

5. Verfahren nach Anspruch 1-3 **gekennzeichnet durch** folgende Verfahrensschritte:
a) Erzeugung überschüssiger ssDNA Amplikons **durch** asymmetrische PCR;
b) Hybridisierung mit einem 5'-FITC markierten Primer, dessen 3'-Ende mit der Position der Mutation korrespondiert;
c) allelspezifische Verlängerung des 5'-FITC markierten Primers **durch** diskriminierende Taq-Polymeraseaktivität mit dNTP's zu partiell einzelsträngigen Duplexen;
d) Immobilisierung von biotinierten genspezifischen Oligonukleotiden auf definierten Spots des Streptavidin beschichteten Glasobjektträgers;
e) Selektive Hybridisierung der partiell einzelsträngigen Duplexe mit den passenden genspezifischen Oligonukleotiden auf den passenden Spots;
f) Entfernung der nicht verlängerten, 5'-FITC markierten Primer **durch** stringente Waschung;
g) Detektion der allelrepräsentierenden FITC markierten Oligonukleotide unter Verwendung eines Anti-FITC-Ak-HRP ELISAs.

6. Verfahren nach Anspruch 1-3, **gekennzeichnet durch** folgende Verfahrensschritte:
a) Amplifizierung von mehreren CYP Allelen in einem Multiplex Ansatz mittels PCR,
b) Überführen der PCR-Produkte aus a) in einen Reaktionsansatz umfaßt **durch**:
- allelspezifische SNP Oligonukleotidsonden, die am 5' Ende markiert sind, bevorzugt Oligonukleotide aus Anlage 2,
- isoformspezifische, 5' phosphorylierte, 3' fluoreszenzmarkierte, Oligonukleotide, deren 5' Ende unmittelbar dem nachzuweisenden, allelspezifischen SNP folgt, vorzugsweise Oligonukleotide aus Anlage 2
- DNA Ligase aus Thermophilus aquaticus,
c) Ligation der allelspezifischen SNP Oligonukleotidsonden mit den isoformspezifischen Oligonukleotiden mit Hilfe der "Taq DNA Ligase detection reaction" Technologie (LDR),
d) SNP Genotypisierung **durch** Detektion der ligierten allelrepräsentierenden 3'-fluoreszenzmarkierten Oligonukleotide,
wobei bevorzugt als allelspezifische SNP Oligonukleotidsonden bzw. als isoformspezifische Oligonukleotide Oligonukleotide aus der Anlage 2 verwendet werden.

7. Verfahren nach Anspruch 6, **dadurch gekennzeichnet, daß** nach der Ligation eine Hybridisierung der entstandenen, 3' fluoreszenzmarkierten LDR Produkte an synthetische Oligonukleotide erfolgt, die zu den Zip Code Sequenzen revers komplementär, 3' Biotin markiert und an Streptavidin beschichteten PC Chips immobilisiert sind, bevorzugt der Nachweis der allelrepräsentierenden 3'-fluoreszenzmarkierten Oligonukleotide in Lösung über dem PC-Chip erfolgt, wobei 3'-FITC markierten Oligonukleotide verwendet werden und eine Anti-FITC-Ak-HRP Kopplung erfolgt, anschließend eine Waschung durchgeführt wird und die Detektion durch kalorimetrischen Nachweis mittels eines eingefüllten TMB Reagenz erfolgt bzw. 3'-Cy5 fluoreszenzmarkierte Oligonukleotide verwendet werden und die Detektion durch direkte Laser enhanced Fluorometrie erfolgt.

8. Testkit zur Durchführung des Verfahrens nach Anspruch 1-5 umfassend folgende Komponenten:
a) Oligonukleotide, die geeignet sind, Genabschnitte in Genen des Arzneimittelmetabolismus, vorzugsweise in Cytochrom P450 Genen (CYP) mit Hilfe der "DNA Polymerase chain reaction" Technologie (PCR) isoformspezifisch (IS-PCR) aus genomischer Leukozyten-DNA zu amplifizieren, vorzugsweise Oligonukleotide gemäß Anlage 1;
b) Komponenten, die es ermöglichen, einzelsträngig biotinierte IS-PCR Produkte auf Streptavidin beschichteten Mikrotiterplatten (SA-MTP) selektiv, thermostabil und einzelsträngig zu immobilisieren,
c) testoptimierte, allelspezifische, Fluoresceinisothiocyanat (FITC) markierte synthetische Oligonukleotide, die durch Hybridisierung, nachfolgende stringente Waschung und anschließende Detektion mittels Fluorometrie bzw. Photometrie eine sichere Genotypisierung der immobilisierten Amplifikationsprodukte (Amplikons) gewährleisten; oder
d) alternativ dazu Streptavidin beschichtete Glasobjektträger (SA-Chip) auf denen biotinierte, isoformspezifische, synthetische Oligonukleotide immobilisiert sind, welche mit partiell einzelsträngigen PCR Produkten hybridisiert werden, die durch diskriminierende Verlängerung eines allelspezifischen, endständig FITC markierten Oligonukleotides mittels Taq DNA Polymerase erzeugt wurden und durch nachfolgende stringente Waschung und anschließende Detektion mittels Fluorometrie bzw. Photometrie eine sichere Genotypisierung der hybridisierten Amplikons gewährleisten.

9. Testkit zur Durchführung des Verfahrens nach Anspruch 1-3 und 6-7 umfassend folgende Komponenten:
a) Oligonukleotide, die geeignet sind, Genabschnitte in Genen des Arzneimittelmetabolismus, vorzugsweise in Cytochrom P450 Genen (CYP) mit Hilfe der "DNA Polymerase chain reaction" Technologie (PCR) isoformspezifisch (IS-PCR) aus genomischer Leukozyten-DNA zu amplifizieren, vorzugsweise Oligonukleotide gemäß Anlage 1 und 2,
b) allelspezifische SNP Oligonukleotidsonden, die am 5' Ende markiert sind, vorzugsweise Oligonukleotide aus Anlage 2,
e) isoformspezifische, 5' phosphorylierte, 3' fluoreszenzmarkierte, Oligonukleotide, deren 5' Ende unmittelbar dem nachzuweisenden, allelspezifischen SNP folgt, vorzugsweise Oligonukleotide aus Anlage 2
c) DNA Ligase.

10. Oligonukleotide und Plasmid DNA Teilsequenzen gemäß Anlage 1 und Anlage 2, sowie deren Varianten, die analoge Eigenschaften aufweisen.
